# FASCICULE DE BREVET EUROPEEN

(11) **EP 1 242 133 B1**
(45) Date de publication et mention de la délivrance du brevet: **09.11.2005**
(21) Numéro de dépôt: 00985305.2
(22) Date de dépôt: 23.11.2000
(51) Int. Cl.: A61M 5/30

(54) **SERINGUE SANS AIGUILLE MUNIE D'UN TUBE D'EJECTION A SECTION CONSTANTE**
NADELLOSE SPRITZE MIT EINEM AUSSTOSSROHR MIT KONSTANTEM QUERSCHNITT
NEEDLESS SYRINGE PROVIDED WITH AN EJECTION TUBE WITH A CONSTANT CROSS-SECTION

(30) Priorité: 08.12.1999 FR 9915474
(43) Date de publication de la demande: 25.09.2002
(73) Titulaire: Crossject, 75004 Paris Cédex 04 (FR)
(72) Inventeur: ALEXANDRE, Patrick, F-70100 Gray (FR); DELANNOY, Guy, F-33160 Saint Médard en Jalles (FR); GAUTIER, Philippe, F-91220 Le Plessis Pate (FR); SIMONIN, Olivier, F-31000 Toulouse (FR)
(86) Numéro de dépôt international: PCT/FR2000/003257
(87) Numéro de publication internationale: WO 2001/041840

(56) Documents cités:
- WO-A-00/54827
- WO-A-00/62846
- WO-A-01/05455
- WO-A-94/24263
- WO-A-96/25190
- US-A- 5 204 253

## Description

Le domaine technique de l'invention est celui des seringues sans aiguille utilisées pour les injections hypodermiques ou intramusculaires de divers principes actifs pulvérulents à usage thérapeutique pour la médecine humaine ou vétérinaire.

Plus spécifiquement, l'invention se rapporte à une seringue sans aiguille mettant en oeuvre un générateur de gaz destiné à créer une onde de pression pour éjecter les particules de principe actif. Un opercule claquable, placé sur le chemin des gaz, permet d'obtenir le niveau de pression seuil permettant d'éjecter les particules avec une vitesse suffisamment élevée. En fait, la libération soudaine des gaz crée dans la seringue un choc thermodynamique et c'est l'onde de choc qui va porter et accélérer les particules afin de les expulser de la seringue. La spécificité de l'invention réside dans le fait que l'effet de l'onde de choc sur les particules de principe actif, est amélioré par la géométrie spécifique du tube d'éjection constituant la partie terminale de la seringue par laquelle sont expulsées lesdites particules.

Les seringues sans aiguille fonctionnant par libération d'un gaz sous pression pour expulser des particules solides de principe actif ont déjà fait l'objet de plusieurs brevets. On peut citer en particulier la demande de brevet WO 94/24263, qui décrit une seringue sans aiguille fonctionnant par libération d'une réserve de gaz, pour entraîner les particules solides de principe actif initialement placées entre deux membranes transversales soufflables, elles-mêmes positionnées dans le tube d'éjection des particules, ledit tube d'éjection présentant une partie amont convergente et une partie aval cylindrique droite ou divergente. La caractéristique de cette seringue est que les deux membranes sont positionnées dans la partie convergente dudit tube, de sorte que la portion du tube située en aval du système de retenue des particules présente d'abord une partie convergente prolongée ensuite par une partie cylindrique droite ou divergente, dont l'extrémité est destinée à venir au contact de la peau du patient à traiter.

L'état de la technique le plus proche est divulgué par le document WO96/25190. Le contenu des documents WO01/05455, WO00/62846 et WO00/54827 est compris dans l'état de la technique selon l'article 54(3) CBE.

Les seringues sans aiguille conçues pour injecter des particules solides de principe actif doivent être peu encombrantes tout en restant très performantes, la notion de performance étant étroitement liée à la vitesse d'expulsion des particules dont la valeur minimum est de l'ordre de 750m/s.

Il demeure particulièrement intéressant de pouvoir améliorer les performances d'une seringue sans aiguille sans avoir à accroître sa source énergétique ou bien ses dimensions. Aussi, dans cette optique, il a été observé qu'une configuration de seringue pour laquelle, d'une part, l'espace libre, appelé aussi chambre d'expansion et situé en aval de la source énergétique, présente un corps cylindrique prolongé par une zone de rétrécissement débouchant dans un tube d'éjection cylindrique de section réduite, et, d'autre part, le moyen de retenue des particules est situé à l'entrée dudit tube en continuité de ladite chambre, permet d'augmenter significativement la vitesse des particules sans avoir à modifier les autres caractéristiques de la seringue. Cette configuration optimisée, pour laquelle une augmentation significative des performances de la seringue a été constatée, se démarque de celle décrite dans le brevet WO 94 24263 par le fait que le moyen de retenue des particules est situé dans le tube d'éjection de section constante et non pas dans la zone de rétrécissement de la chambre d'expansion.

Les seringues sans aiguille selon l'invention présente ce perfectionnement.

L'objet de la présente invention concerne une seringue sans aiguille comprenant successivement un générateur de gaz, une chambre d'expansion des gaz, un moyen de retenue des particules d'un principe actif comprenant au moins un opercule destiné à se rompre sous l'effet des gaz provenant dudit générateur, et un tube d'éjection desdites particules, ledit tube présentant une partie amont cylindrique dans laquelle est fixé le moyen de retenue des particules, ladite partie amont ayant une section sensiblement constante sur une longueur supérieure à deux fois son diamètre interne. Le générateur de gaz de la seringue sans aiguille selon l'invention est un générateur de gaz pyrotechnique impliquant une charge pyrotechnique et son système d'initiation.

Avantageusement, la chambre d'expansion des gaz est prolongée par ladite partie amont du tube et l'opercule, qui est l'élément le plus en amont du moyen de retenue des particules, constitue, de façon précise, la limite entre ladite chambre et ledit tube.

De façon avantageuse, l'opercule est calibré pour céder à une pression dynamique dans la chambre d'au moins 70 bars et, préférentiellement, à une pression dynamique comprise entre 80 bars et 200 bars. Avantageusement, l'opercule est préfragmenté, de sorte qu'il est destiné à s'ouvrir en pétales sous l'effet de la poussée des gaz, sans émettre de particules.

Le tube d'éjection est un cylindre droit sur toute sa longueur.

Selon un premier mode de réalisation préféré de l'invention, la chambre d'expansion des gaz est sensiblement cylindrique et son diamètre interne est voisin de celui du tube d'éjection. Préférentiellement, les diamètres de la chambre d'expansion et du tube d'éjection sont égaux à 12 mm. De façon avantageuse, le rapport de la somme des longueurs de la chambre et du tube sur leur diamètre est compris entre 3 et 25, et préférentiellement entre 7 et 18.

Selon un second mode de réalisation préféré de l'invention, la chambre d'expansion présente une forme sensiblement cylindrique prolongée par une zone de rétrécissement débouchant sur le tube d'éjection, de sorte que le diamètre interne dudit tube est inférieur au diamètre interne de la partie cylindrique de ladite chambre et l'opercule est fixé à l'entrée du tube d'éjection de diamètre réduit.

De façon préférentielle, la zone de rétrécissement est progressive en ayant une forme de convergent de tuyère. En effet, le passage d'une configuration où les diamètres de la chambre et du tube sont identiques à une configuration pour laquelle le diamètre du tube est inférieur à celui de la chambre, s'accompagne toujours d'un accroissement des vitesses d'éjection des particules de principe actif, et ce, pour une même source énergétique. Il peut également être envisagé une zone de rétrécissement brusque, matérialisée par un épaulement interne marquant une cassure nette entre la chambre et le tube d'éjection.

De façon avantageuse, le rapport du diamètre de la partie cylindrique de la chambre d'expansion sur le diamètre interne du tube d'éjection est compris entre 1,1 et 3, et préférentiellement entre 1,5 et 2.

Selon l'un ou l'autre des deux modes de réalisation préférés de l'invention précédents, le rapport de la longueur du tube sur la longueur de la chambre est compris entre 1 et 5 et la somme de ces deux longueurs est comprise entre 8 cm et 15 cm. Préférentiellement, la longueur de la chambre vaut 3,5 cm et celle du tube vaut 8,5 cm.

Avantageusement, le diamètre des particules de principe actif est compris entre 20µm et 100µm et préférentiellement entre 50µm et 80µm, et la masse totale dudit principe actif est comprise entre 1 mg et 10 mg et préférentiellement entre 2 mg et 7 mg. De façon avantageuse, les particules sont logées entre l'opercule et une membrane placée en aval dudit opercule. Préférentiellement, ladite membrane est fine, inélastique et transversale par rapport à l'axe du tube et présente des lignes de fragilisation pour s'ouvrir également en pétales. Avantageusement, le compactage des particules est compris entre 1% et 70%, et préférentiellement entre 10% et 50%. Le compactage se définit comme étant le rapport du volume total des particules sur le volume total du tube d'éjection compris entre l'opercule et la membrane.

De façon préférentielle, la densité des particules de principe actif est comprise entre 1 et 18, et préférentiellement entre 3 et 10. En fait, c'est la combinaison des deux paramètres « diamètre des particules » et « densité des particules » qui va conditionner leur vitesse d'éjection. En théorie, la vitesse des particules est inversement proportionnelle à la densité et au carré du diamètre. Par calcul et par essais, il a été montré que des particules de petit diamètre peuvent avoir des densités élevées sans pour autant affecter, de façon significative, leur vitesse. En revanche, si les particules ont une taille importante tout en ayant une densité également importante, le risque à redouter est que l'onde de choc, issue de l'opercule qui se déchire, traverse ces particules à forte inertie, sans véritablement les porter tout le long de leur trajet, avec pour conséquence majeure une décélération des particules par rapport aux gaz d'entraînement, et en définitive, une vitesse d'impact sur la peau trop faible pour permettre leur pénétration.

De façon avantageuse, la partie amont cylindrique du tube d'éjection présente une section sensiblement constante sur une longueur supérieure à trois fois et demi son diamètre interne.

Les seringues sans aiguille selon l'invention présentent l'avantage de posséder un niveau de performance accru, en terme de vitesse d'expulsion des particules, sans avoir à augmenter ni leur source énergétique ni leur encombrement.

De plus, la géométrie particulière du tube d'éjection appartenant aux seringues sans aiguille selon l'invention, reste très simple et ne nécessite donc pas un usinage sophistiqué, long et coûteux.

Enfin, les seringues sans aiguille selon l'invention demeurent aussi efficaces, qu'elles fonctionnent avec un générateur de gaz pyrotechnique.

On donne, ci-après, la description détaillée de deux modes de réalisation préférés de l'invention en se référant aux figures.

La figure 1 est une vue en coupe axiale longitudinale d'une seringue sans aiguille selon l'invention pour laquelle les diamètres de la chambre d'expansion et du tube d'éjection sont identiques.

La figure 2 est un schéma en coupe axiale longitudinale d'une chambre d'expansion présentant une zone de rétrécissement prolongée par un tube d'éjection cylindrique droit.

La figure 3, qui ne fait pas partie de l'invention, est un schéma en coupe axiale longitudinale d'une chambre d'expansion analogue à celle de la figure 2 pour laquelle le tube d'éjection présente une partie terminale divergente.

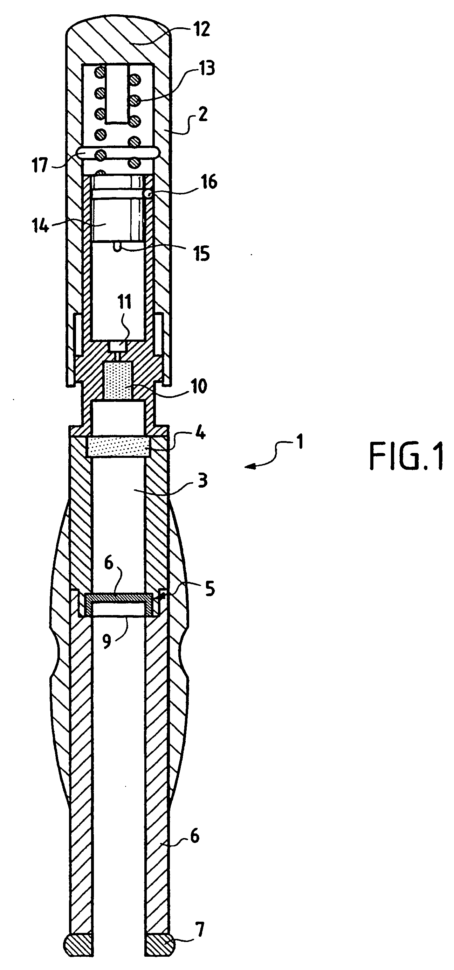

En se référant à la figure 1, une seringue sans aiguille 1 selon l'invention comprend successivement un générateur de gaz pyrotechnique 2, une chambre d'expansion 3 munie d'un filtre 4, un système de retenue 5 des particules et le tube d'éjection 6 desdites particules destiné à venir en appui contre la peau du patient à traiter.

Préférentiellement, cette mise en appui peut être facilitée par un bourrelet 7 amortissant situé à l'extrémité dudit tube 6. La chambre d'expansion 3 des gaz ainsi que le canal interne du tube d'éjection 6 sont sensiblement de forme cylindrique et ont tous les deux le même diamètre. Le système de retenue 5 des particules qui marque la frontière entre la chambre 3 et le tube 6 est constitué par un opercule claquable 8 et une membrane légère 9 placée en aval dudit opercule, ces deux éléments étant parallèles entre eux, en position transversale par rapport à l'axe du tube 6 et fixés tous les deux audit tube 6. Les particules de principe actif occupent l'espace délimité par ces deux éléments, avec un taux de compactage préférentiellement compris entre 1% et 70%.

Selon un mode de réalisation préféré de l'invention, la longueur de la chambre 3 est de 3,5 cm, la longueur du tube d'éjection 6 est de 8,5 cm et leur diamètre vaut 0,8 cm. L'opercule claquable, qui est situé du côté de la chambre d'expansion 3, est calibré pour se rompre à une pression dynamique au moins égale à 70 bars et la membrane 9, quant à elle, sert uniquement de maintien aux particules, sans présenter le moindre caractère de résistance vis à vis des gaz produits.

Avantageusement, ladite membrane 9 est fine et inélastique et présente, comme l'opercule 8, des lignes d'affaiblissement définissant un motif étoilé pour permettre une ouverture en pétales, sans risquer de provoquer un morcellement désordonné pouvant conduire à la production de morceaux parasites.

Selon un autre mode de réalisation, la membrane 9 peut être remplacée par une grille transversale également fixée à l'intérieur du tube 6 et contenant, insérées dans ses interstices, les particules de principe actif. Par rapport au sens de propagation des gaz émis, l'opercule 8 reste en amont de ladite grille.

La chambre d'expansion 3 possède à son extrémité la plus proche du générateur pyrotechnique 2 de gaz un filtre 4 transversal destiné, d'une part, à piéger certaines particules solides issues de la combustion et, d'autre part, à refroidir les gaz avant qu'ils n'entrent dans ladite chambre 3. Avantageusement, ledit filtre 4 est constitué d'un empilement de grilles métalliques avec un pas de plus en plus rapproché et se terminant par une feuille de papier céramique.

Ce filtre 4 permet à la température des gaz de ne pas excéder 1500 K dans la chambre d'expansion 3, de façon à ne pas altérer les particules de principe actif disposées dans leur logement. Le générateur pyrotechnique 2 de gaz comprend un dispositif d'initiation de la charge pyrotechnique 10 faisant intervenir un dispositif de percussion et une amorce 11. Le dispositif de percussion, qui est déclenché par un bouton poussoir 12, comprend un ressort 13 et une masselotte 14 munie d'un percuteur 15. La masselotte 14 est bloquée par au moins une bille 16 coincée entre ladite masselotte 14 et le bouton poussoir 12 et ledit bouton poussoir 12 possède une gorge interne 17 circulaire.

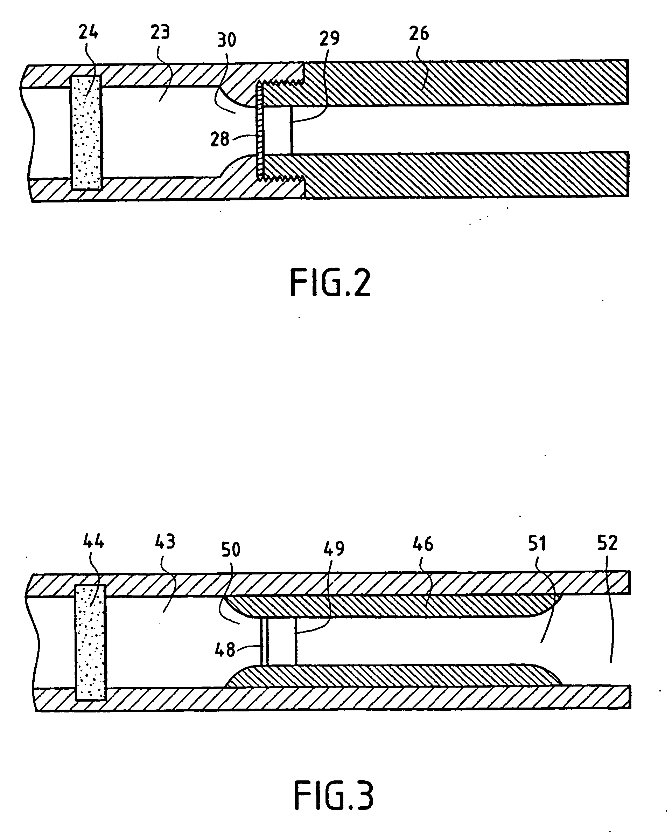

En se référant à la figure 2, selon un deuxième mode de réalisation préféré de l'invention, la seringue sans aiguille comprend successivement un générateur pyrotechnique de gaz non représenté sur la figure, une chambre d'expansion 23 des gaz, un moyen de retenue des particules constitué également par un opercule claquable 28 et une membrane 29 placée en aval dudit opercule, et un tube d'éjection 26 desdites particules. La chambre 23 possède un filtre 24 ayant les mêmes fonctions que celles décrites pour le premier mode de réalisation préféré de l'invention, à savoir, capture des particules solides indésirables et refroidissement des gaz de combustion.

Selon ce mode de réalisation préféré de l'invention, la seringue possède le même générateur de gaz pyrotechnique que celui décrit succinctement pour le premier mode de réalisation préféré de l'invention. La principale différence avec le premier mode de réalisation ci-avant décrit, réside dans le fait que le tube d'éjection 26 a un diamètre interne réduit, inférieur à celui de la chambre d'expansion 23. Plus précisément, la chambre d'expansion 23 présente une forme sensiblement cylindrique prolongée par une zone de rétrécissement 30 progressive débouchant sur le tube d'éjection 26 à l'entrée duquel les particules de principe actif sont logées, entre l'opercule 28 et la membrane 29.

Selon le deuxième mode de réalisation préféré de l'invention, plusieurs configurations ont été étudiées, et notamment celles pour lesquelles la zone de rétrécissement est progressive en prenant la forme d'un convergent de tuyère, et celles pour lesquelles la zone de rétrécissement est brusque, matérialisée par un épaulement interne. Le tableau 1 ci-après résume les configurations étudiées.

**TABLEAU 1 :**

| configurations étudiées. | |
|---|---|
| CONFIGURATION | DESCRIPTION |
| Nominal | - Øchambre = 8mm |
| | - Øtube = 8 mm |
| Dcc 12 | - Øchambre = 12 mm |
| | - Øtube = 8 mm |
| Dcc 16 | - Øchambre = 16 mm |
| | - Øtube = 8 mm |
| | - zone de rétrécissement : brusque |
| Cvg D12 | - Øchambre = 12 mm |
| | - Øtube = 8 mm |
| | - zone de rétrécissement : progressive |
| Cvg D12-Opt - | - Øchambre = 12 mm |
| | Øtube = 8 mm |
| | - zone de rétrécissement : progressive |
| | - configuration optimisée |
| Cvg D16-Opt - | - Øchambre = 16 mm |
| | Øtube = 8 mm |
| | - zone de rétrécissement : progressive |
| | - configuration optimisée |

- Dcc =: zone de rétrécissement brusque : un épaulement interne marque la limite entre la chambre et le tube
- Cvg =: zone de rétrécissement progressive en forme de convergent de tuyère
- Opt =: configuration optimisée = configuration pour laquelle la somme de la longueur de la chambre et du tube est égale à 10 cm
- Nominal =: la chambre et le tube ont le même diamètre.

La courbe 1, ci-après, donne les résultats obtenus en terme de vitesse des gaz et des particules en sortie de tube, pour chaque configuration étudiée.

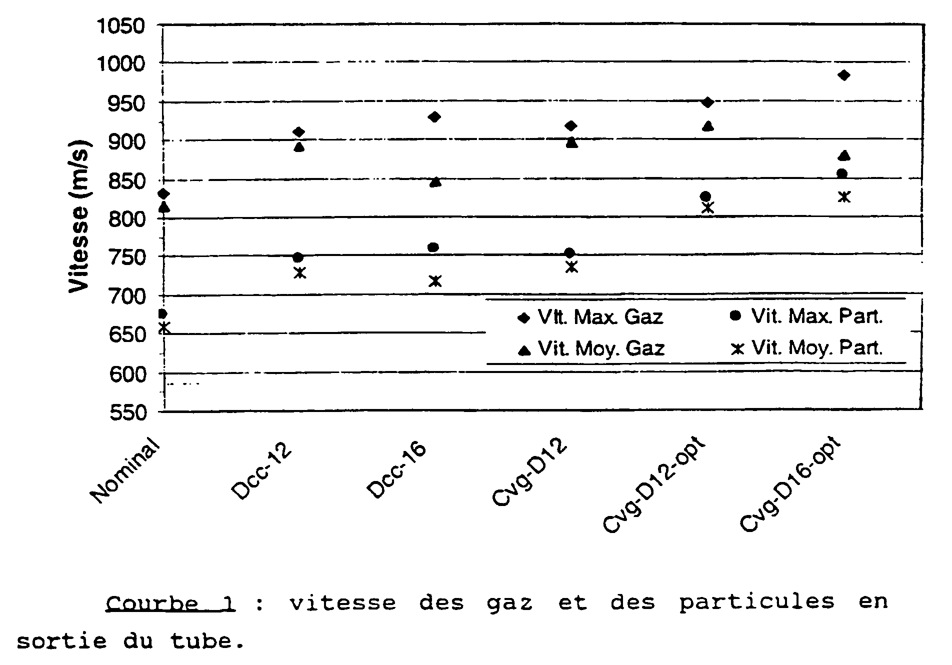

Le tableau 2 ci-après, présente pour chaque configuration, les écarts relatifs de vitesse moyenne des particules, entre les configurations étudiées et la configuration nominale.

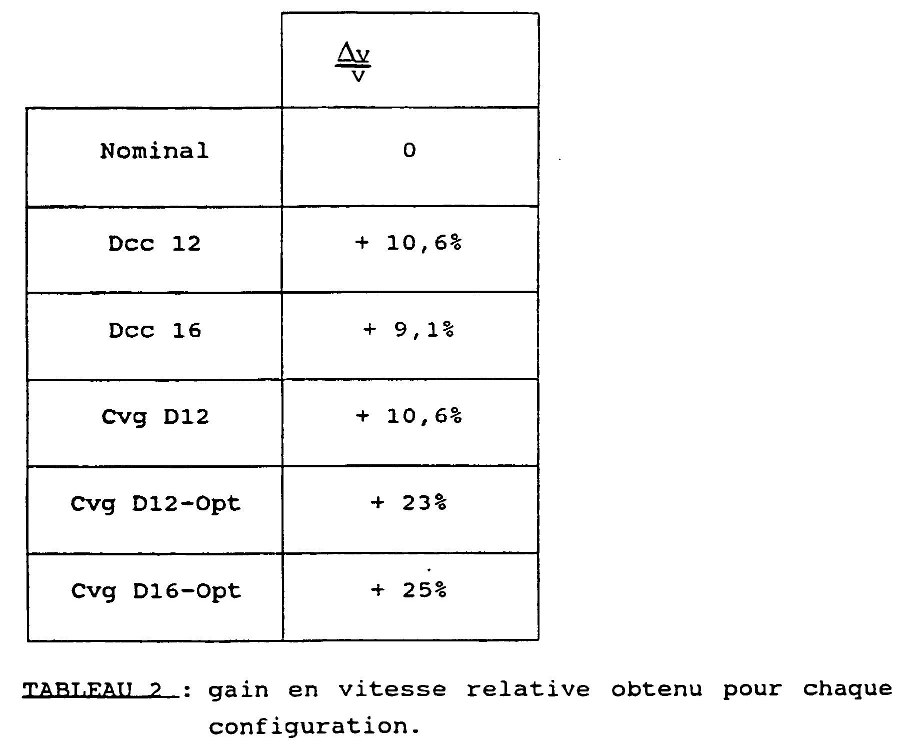

- Δv =: différence entre la vitesse obtenue pour la configuration considérée et celle obtenue pour la configuration nominale
- v =: vitesse nominale

Ces résultats montrent clairement que, quelle que soit la forme de la zone de rétrécissement de la chambre d'expansion, qu'elle soit brusque ou progressive, il y a un gain de la vitesse des particules en sortie de tube d'éjection par rapport à la configuration nominale. De plus, il est important de souligner que ce gain est significatif puisqu'il peut atteindre une valeur de 25% pour une configuration optimisée.

Préférentiellement, la somme des longueur de la chambre 23 et du tube 26 vaut 10 cm et les diamètres de la chambre 23 et du tube 26 valent respectivement 1,2 cm et 0,8 cm.

Préférentiellement, la zone de rétrécissement 30 a la forme d'un convergent de tuyère et sa longueur vaut 0,6 cm. Pour un générateur de gaz pyrotechnique donné, la configuration pour laquelle la section du tube 26 est inférieure à celle de la chambre 23 est plus performante, en terme de vitesse d'émission de particules de principe actif, que celle pour laquelle la chambre 3 et le tube 6 sont en continuité l'un de l'autre avec le même diamètre.

En se référant à la figure 3, qui ne fait pas partie de l'invention, la seringue sans aiguille comprend successivement un générateur pyrotechnique de gaz non représenté sur la figure, une chambre d'expansion 43 des gaz, un moyen de retenue des particules constitué également par un opercule claquable 48 et une membrane 49, et un tube d'éjection 46 desdites particules. La chambre 43 possède un filtre 44 ayant les mêmes fonctions que celles décrites précédemment. Le tube d'éjection 46 a un diamètre réduit inférieur à celui de la chambre d'expansion 43, ladite chambre 43 présentant une forme sensiblement cylindrique prolongée par une zone de rétrécissement 50 progressive débouchant sur le tube d'éjection 46. Le système de retenue des particules est situé au même endroit que celui précisé dans la description du deuxième mode de réalisation préféré de l'invention. La différence fondamentale avec le deuxième mode de réalisation préféré de l'invention est que le segment aval du tube 46 par lequel sont éjectées les particules présente une partie conique divergente 51 prolongée par une partie cylindrique droite 52 dont l'extrémité libre vient au contact de la peau du patient à traiter. Cet évasement terminal constitue un évent pour la surpression produite dans le tube 46 et a pour fonction principale de disperser la pression résiduelle en sortie de seringue, de façon à diminuer toute sollicitation indésirable pouvant être néfaste pour le patient. Cette chute de pression n'affecte pratiquement pas la vitesse des particules au moment où elles vont impacter la peau.

Les caractéristiques dimensionnelles du deuxième mode de réalisation de l'invention sont conservées, en précisant en plus que la longueur de la zone conique divergente 51 du tube 46 vaut approximativement 0,8 cm.

Avantageusement, la partie cylindrique droite 52 prolongeant la partie conique divergente 51 dudit tube 46 a un diamètre identique à celui de la partie cylindrique de la chambre 43, ledit diamètre valant approximativement 1,2 cm. Une telle configuration, par rapport à la même configuration sans partie terminale divergente, permet de faire chuter la pression de 35% en sortie du tube 46.

## Revendications

1. Seringue sans aiguille comprenant successivement un générateur de gaz (2), une chambre (3, 23) d'expansion des gaz, un moyen de retenue des particules d'un principe actif comprenant au moins un opercule destiné à se rompre sous l'effet des gaz provenant dudit générateur, et un tube d'éjection (6, 26) desdites particules, ledit tube (6, 26) présentant une partie amont cylindrique dans laquelle est fixé le moyen de retenue des particules, ladite partie amont ayant une section sensiblement constante sur une longueur supérieure à deux fois son diamètre interne, **caractérisé en ce que** le générateur de gaz (2) est un générateur pyrotechnique impliquant une charge pyrotechnique (10) et un dispositif d'initiation de ladite charge et **en ce que** le tube d'éjection est un cylindre droit sur toute sa longueur.

2. Seringue sans aiguille selon la revendication 1, **caractérisée en ce que** la chambre d'expansion (3, 23) est prolongée par ladite partie amont du tube (6, 26) et l'opercule (8, 28) qui est l'élément le plus en amont du moyen de retenue des particules constitue la limite entre ladite chambre (3, 23) et ledit tube (6, 26).

3. Seringue sans aiguille selon la revendication 2, **caractérisée en ce que** l'opercule (8, 28) est calibré pour céder à une pression dynamique dans la chambre (3, 23) d'au moins 70 bars.

4. Seringue sans aiguille selon la revendication 3, **caractérisée en ce que** la chambre d'expansion (3) des gaz est sensiblement cylindrique et son diamètre interne est voisin de celui du tube d'éjection (6).

5. Seringue sans aiguille selon la revendication 4, **caractérisée en ce que** le rapport de la somme des longueurs de la chambre (3) et du tube (6) sur leur diamètre est compris entre 3 et 25.

6. Seringue sans aiguille selon la revendication 2, **caractérisée en ce que** la chambre d'expansion (23) présente une forme sensiblement cylindrique prolongée par une zone de rétrécissement (30) débouchant sur le tube d'éjection (26), de sorte que le diamètre interne dudit tube (26) est inférieur au diamètre interne de la partie cylindrique de ladite chambre (23) et l'opercule (28) est fixé dans le tube d'éjection (26) de diamètre réduit.

7. Seringue sans aiguille selon la revendication 6, **caractérisée en ce que** la zone de rétrécissement (30) est progressive en ayant une forme de convergent de tuyère.

8. Seringue sans aiguille selon la revendication 6, **caractérisée en ce que** le rapport du diamètre de la partie cylindrique de la chambre d'expansion (23) sur le diamètre interne du tube d'éjection (26) est compris entre 1,1 et 3.

9. Seringue sans aiguille selon la revendication 6, **caractérisée en ce que** le rapport du diamètre de la partie cylindrique de la chambre d'expansion (23) sur le diamètre interne du tube d'éjection (26) est compris entre 1,5 et 2.

10. Seringue sans aiguille selon l'une quelconque des revendications 1, 2, 4, ou 6, **caractérisée en ce que** le rapport de la longueur du tube (6, 26) sur la longueur de la chambre (3, 23) est compris entre 1 et 5 et la somme de ces deux longueurs est comprise entre 8 cm et 15 cm.

11. Seringue sans aiguille selon la revendication 1, **caractérisée en ce que** le diamètre des particules de principe actif est compris entre 20µm et 100µm et la masse totale dudit principe actif est comprise entre 1 mg et 10 mg.

12. Seringue sans aiguille selon l'une quelconque des revendications 1, 2 ou 11, **caractérisée en ce que** les particules sont logées entre l'opercule (8, 28) et une membrane (9, 29) placée en aval dudit opercule (8, 28).

13. Seringue sans aiguille selon l'une quelconque revendications 1, 2, 11 ou 12, **caractérisée en ce que** le compactage des particules est compris entre 1% et 70%.

14. Seringue sans aiguille selon la revendication 1, **caractérisée en ce que** la densité des particules est comprise entre 1 et 18.

15. Seringue sans aiguille selon l'une quelconque revendications 1, 2, 4, 5 ou 6, **caractérisée en ce que** la partie amont cylindrique du tube d'éjection (6, 26) présente une section sensiblement constante sur une longueur supérieure à trois fois et demi son diamètre interne.

16. Seringue sans aiguille selon la revendication 4, **caractérisée en ce que** les diamètres de la chambre d'expansion (3) et du tube d'éjection (6) sont égaux à 12 mm.

17. Seringue sans aiguille selon la revendication 1, **caractérisé en ce que** le dispositif d'initiation de la charge pyrotechnique (10) comprend un dispositif de percussion et une amorce (11).

18. Seringue sans aiguille selon la revendication 1, **caractérisé en ce que** la chambre d'expansion (3) possède un filtre (4) pour piéger les particules issues de la combustion et pour refroidir les gaz.

19. Seringue sans aiguille selon la revendication 18, **caractérisé en ce que** le filtre (4) est constitué d'un empilement de grilles métalliques avec un pas de plus en plus rapproché et se terminant par une feuille de papier céramique.

## Patentansprüche

1. Nadellose Spritze, die nacheinander einen Gasgenerator (2), einen Expansionsraum (3, 23) für die Gase, ein Rückhaltemittel für die Teilchen eines Wirkstoffs, das mindestens eine Abdeckfolie aufweist, die dazu bestimmt ist, unter der Wirkung der vom Generator kommenden Gase zu reißen, und ein Ausstoßrohr (6, 26) der Teilchen aufweist, wobei das Rohr (6, 26) einen vorderen zylindrischen Bereich aufweist, in dem das Mittel zum Zurückhalten der Teilchen befestigt ist, wobei der vordere Bereich über eine Länge, die größer ist als sein doppelter Innendurchmesser, einen im Wesentlichen konstanten Querschnitt hat, **dadurch gekennzeichnet, dass** der Gasgenerator (2) ein pyrotechnischer Generator ist, der eine pyrotechnische Ladung (10) und eine Zündvorrichtung für die Ladung einschließt, und dass das Ausstoßrohr ein über seine ganze Länge gerader Zylinder ist.

2. Nadellose Spritze nach Anspruch 1, **dadurch gekennzeichnet, dass** der Expansionsraum (3, 23) vom vorderen Bereich des Rohrs (6, 26) verlängert wird, und die Abdeckfolie (8, 28), die das am weitesten vorne liegende Element des Mittels für den Rückhalt der Teilchen ist, die Grenze zwischen der Kammer (3, 23) und dem Rohr (6, 26) bildet.

3. Nadellose Spritze nach Anspruch 2, **dadurch gekennzeichnet, dass** die Abdeckfolie (8, 28) so bemessen ist, dass sie einem dynamischen Druck in der Kammer (3, 23) von mindestens 70 Bar nachgibt.

4. Nadellose Spritze nach Anspruch 3, **dadurch gekennzeichnet, dass** der Expansionsraum (3) für die Gase im Wesentlichen zylindrisch und sein Innendurchmesser ähnlich dem des Ausstoßrohrs (6) ist.

5. Nadellose Spritze nach Anspruch 4, **dadurch gekennzeichnet, dass** das Verhältnis der Summe der Längen der Kammer (3) und des Rohrs (6) zu ihrem Durchmesser zwischen 3 und 25 liegt.

6. Nadellose Spritze nach Anspruch 2, **dadurch gekennzeichnet, dass** der Expansionsraum (23) eine im Wesentlichen zylindrische Form aufweist, die von einer Verengungszone (30) verlängert wird, die an dem Ausstoßrohr (26) mündet, so dass der Innendurchmesser des Rohrs (26) kleiner ist als der Innendurchmesser des zylindrischen Bereichs der Kammer (23), und die Abdeckfolie (28) im Ausstoßrohr (26) mit verringertem Durchmesser befestigt ist.

7. Nadellose Spritze nach Anspruch 6, **dadurch gekennzeichnet, dass** die Verengungszone (30) progressiv ist und die Form einer konvergenten Düse aufweist.

8. Nadellose Spritze nach Anspruch 6, **dadurch gekennzeichnet, dass** das Verhältnis zwischen dem Durchmesser des zylindrischen Bereichs des Expansionsraums (23) und dem Innendurchmesser des Ausstoßrohrs (26) zwischen 1,1 und 3 liegt.

9. Nadellose Spritze nach Anspruch 6, **dadurch gekennzeichnet, dass** das Verhältnis zwischen dem Durchmesser des zylindrischen Bereichs des Expansionsraums (23) und dem Innendurchmesser des Ausstoßrohrs (26) zwischen 1,5 und 2 liegt.

10. Nadellose Spritze nach einem der Ansprüche 1, 2, 4 oder 6, **dadurch gekennzeichnet, dass** das Verhältnis zwischen der Länge des Rohrs (6, 26) und der Länge des Raums (3, 23) zwischen 1 und 5 und die Summe dieser beiden Längen zwischen 8 cm und 15 cm liegt.

11. Nadellose Spritze nach Anspruch 1, **dadurch gekennzeichnet, dass** der Durchmesser der Wirkstoffteilchen zwischen 20 µm und 100 µm und die Gesamtmasse des Wirkstoffs zwischen 1 mg und 10 mg liegt.

12. Nadellose Spritze nach einem der Ansprüche 1, 2 oder 11, **dadurch gekennzeichnet, dass** die Teilchen zwischen der Abdeckfolie (8, 28) und einer Membran (9, 29) untergebracht sind, die hinter der Abdeckfolie (8, 28) liegt.

13. Nadellose Spritze nach einem der Ansprüche 1, 2, 11 oder 12, **dadurch gekennzeichnet, dass** die Verdichtung der Teilchen zwischen 1 % und 70 % liegt.

14. Nadellose Spritze nach Anspruch 1, **dadurch gekennzeichnet, dass** die Dichte der Teilchen zwischen 1 und 18 liegt.

15. Nadellose Spritze nach einem der Ansprüche 1, 2, 4, 5 oder 6, **dadurch gekennzeichnet, dass** der vordere zylindrische Bereich des Ausstoßrohrs (6, 26) über eine Länge, die größer ist als das Dreieinhalbfache seines Innendurchmessers, einen im Wesentlichen konstanten Querschnitt aufweist.

16. Nadellose Spritze nach Anspruch 4, **dadurch gekennzeichnet, dass** die Durchmesser des Expansionsraums (3) und des Ausstoßrohrs (6) 12 mm betragen.

17. Nadellose Spritze nach Anspruch 1, **dadurch gekennzeichnet, dass** die Zündvorrichtung der pyrotechnischen Ladung (10) eine Schlagvorrichtung und einen Zünder (11) aufweist.

18. Nadellose Spritze nach Anspruch 1, **dadurch gekennzeichnet, dass** der Expansionsraum (3) ein Filter (4) aufweist, um die von der Verbrennung kommenden Teilchen einzufangen und um die Gase zu kühlen.

19. Nadellose Spritze nach Anspruch 18, **dadurch gekennzeichnet, dass** das Filter (4) aus einer Stapelung von Metallgittern mit einem immer enger werdenden Abstand besteht und in einem Blatt aus Keramikpapier endet.

## Claims

1. A needleless syringe comprising, in succession, a gas generator (2), a gas-expansion chamber (3, 23), a means for holding back the particles of an active ingredient, said means comprising at least one seal which is intended to rupture under the influence of the gases coming from said generator, and a tube (6, 26) for ejection of said particles, said tube (6, 26) exhibiting a cylindrical upstream part in which the means for holding back the particles is fixed, said upstream part having an approximately constant cross-section over a length greater than twice its internal diameter, **characterised in that** the gas generator (2) is a pyrotechnic generator involving a pyrotechnic charge (10) and a device for initiating said charge, and **in that** the ejection tube is a right cylinder over its entire length.

2. Needleless syringe according to Claim 1,
**characterised in that** the expansion chamber (3, 23) is extended by said upstream part of the tube (6, 26), and the seal (8, 28), which is the most upstream element of the means for holding back the particles, constitutes the boundary between said chamber (3, 23) and said tube (6, 26).

3. Needleless syringe according to Claim 2,
**characterised in that** the seal (8, 28) is gauged so as to give way at a dynamic pressure in the chamber (3, 23) of at least 70 bar.

4. Needleless syringe according to Claim 3,
**characterised in that** the expansion chamber (3) of the gases is approximately cylindrical and its internal diameter is close to that of the ejection tube (6).

5. Needleless syringe according to Claim 4,
**characterised in that** the ratio of the sum of the lengths of the chamber (3) and of the tube (6) to their internal diameter is between 3 and 25.

6. Needleless syringe according to Claim 2,
**characterised in that** the expansion chamber (23) exhibits an approximately cylindrical shape which is extended by a narrowed zone (30) which opens into the ejection tube (26), so that the internal diameter of the cylindrical part of said tube (26) is less than the internal diameter of the cylindrical part of said chamber (23), and the seal (28) is fixed in the ejection tube (26) of reduced diameter.

7. Needleless syringe according to Claim 6,
**characterised in that** the narrowed zone (30) is progressive, having a convergent nozzle shape.

8. Needleless syringe according to Claim 6,
**characterised in that** the ratio of the diameter of the cylindrical part of the expansion chamber (23) to the internal diameter of the ejection tube (26) is between 1.1 and 3.

9. Needleless syringe according to Claim 6,
**characterised in that** the ratio of the diameter of the cylindrical part of the expansion chamber (23) to the internal diameter of the ejection tube (26) is between 1.5 and 2.

10. Needleless syringe according to any one of Claims 1, 2, 4 or 6, **characterised in that** the ratio of the length of the tube (6, 26) to the length of the chamber (3, 23) is between 1 and 5 and the sum of these two lengths is between 8 cm and 15 cm.

11. Needleless syringe according to Claim 1,
**characterised in that** the diameter of the particles of active ingredient is between 20 µm and 100 µm and the total mass of said active ingredient is between 1 mg and 10 mg.

12. Needleless syringe according to any one of Claims 1, 2 or 11, **characterised in that** the particles are accommodated between the seal (8, 28) and a membrane (9, 29) placed downstream of said seal (8, 28).

13. Needleless syringe according to any one of Claims 1, 2, 11 or 12, **characterised in that** the compaction of the particles is between 1 % and 70%.

14. Needleless syringe according to Claim 1,
**characterised in that** the relative density of the particles is between 1 and 18.

15. Needleless syringe according to any one of Claims 1, 2, 4, 5 or 6, **characterised in that** the cylindrical upstream part of the ejection tube (6, 26) exhibits an approximately constant cross-section over a length greater than three-and-a-half times its internal diameter.

16. Needleless syringe according to Claim 4,
**characterised in that** the internal diameters of the expansion chamber (3) and of the ejection tube (6) are equal to 12 mm.

17. Needleless syringe according to Claim 1,
**characterised in that** the device for initiating the pyrotechnic charge (10) comprises a percussion device and a detonator (11).

18. Needleless syringe according to Claim 1,
**characterised in that** the expansion chamber (3) possesses a filter (4) for trapping the particles resulting from combustion and for cooling the gases.

19. Needleless syringe according to Claim 18,
**characterised in that** the filter (4) is constituted by a stack of metallic gratings having an increasingly close spacing and terminated by a sheet of ceramic paper.
